(19) Europäisches Patentamt
European Patent Office
Office européen des brevets

(11) Veröffentlichungsnummer: **0 238 927**
**B1**

(12) # EUROPÄISCHE PATENTSCHRIFT

(45) Veröffentlichungstag der Patentschrift: **31.10.90**

(51) Int. Cl.⁵: **A61K 7/06**, A61K 7/48

(21) Anmeldenummer: **87103354.4**

(22) Anmeldetag: **09.03.87**

(54) **Sebosuppressive Zubereitungen.**

(30) Priorität: **17.03.86 DE 3608852**

(43) Veröffentlichungstag der Anmeldung:
**30.09.87 Patentblatt 87/40**

(45) Bekanntmachung des Hinweises auf die Patenterteilung:
**31.10.90 Patentblatt 90/44**

(84) Benannte Vertragsstaaten:
**AT BE CH DE ES FR GB IT LI NL SE**

(56) Entgegenhaltungen:
**EP-A- 0 114 051**
**BE-A- 903 646**
**DE-A- 3 500 972**
**FR-A- 2 437 207**
**US-A- 4 141 976**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Möller, Hinrich, Dr., Schumannstrasse 11, D-4019 Monheim(DE)**
Erfinder: **Wallat, Siegfried, Dr., Marie-Curie-Strasse 9, D-4019 Monheim(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

"Sebosuppressive Zubereitungen"

Gegenstand der Erfindung sind topisch anzuwendende Zubereitungen zur Verringerung des fettigen, unästhetischen Aussehens der Haare und der Haut, welche eine Kombination aus 4-Alkoxy- oder 4-(4-Alkylbenzyloxy)-benzoesäuren und Purinverbindungen als antiseborrhoischen Wirkstoff enthalten.

Starke Absonderungen der Talgdrüsen der Kopfhaut verursachen ein fettiges Aussehen der Haare, das im allgemeinen als wenig ästhetisch empfunden wird. Auch neigen bestimmte Hautpartien zu übermäßiger Talgdrüsenproduktion, was einem kosmetisch sehr unbefriedigenden Zustand verursacht. Die moderne Kosmetik ist daher bemüht, durch geeignete Zubereitungen die Sekretion der Talgdrüsen zu normalisieren und dem Haar und der Haut wieder ein gesundes und ansprechendes Aussehen zu verleihen.

Obwohl bereits eine große Zahl von antiseborrhoisch wirksamen synthetischen Produkten vorgeschlagen wurde, besteht weiterhin ein Bedürfnis an Zubereitungen mit einer erhöhten Wirksamkeit bei niedrigen Anwendungskonzentrationen. Aufgabe der Erfindung ist es, antiseborrhoische Zubereitungen bereitzustellen, die gegenüber bekannten Präparaten eine verstärkte Wirkung, ohne nachteilige Nebenwirkungen auf den menschlichen Körper, haben.

4-Alkoxybenzoesäuren und deren physiologisch verträgliche Salze sind bereits in der DE-A1-35 00 972 als antiseborrhoische Zusätze für kosmetische Zubereitungen vorgeschlagen worden. Es wurde nun gefunden, daß sich die Wirksamkeit dieser Verbindungen durch Kombination mit selbst nicht antiseborrhoisch wirksamen Purinverbindungen synergistisch steigern läßt. Durch diesen Effekt der erfindungsgemäßen Kombination läßt sich die wirksame Dosierung der Produkte erheblich senken.

Gegenstand der Erfindung sind sebosuppressive Zubereitungen zur topischen Anwendung auf dem Haar und der Haut, bestehend aus anitseborrhoischen Wirkstoffen und einem kosmetischen Träger, dadurch gekennzeichnet, daß als antiseborrhoische Wirkstoffe 4-Alkoxy- oder 4-(4-Alkylbenzyloxy)-benzoesäuren der Formel I

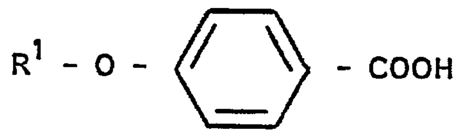

(I)

in der $R^1$ eine Alkylgruppe mit 6 bis 18 C-Atomen oder eine 4-Alkylbenzylgruppe mit 4 bis 12 C-Atomen in der Alkylgruppe ist oder deren physiologisch verträgliche Salze und Purinverbindungen der allgemeinen Formel II

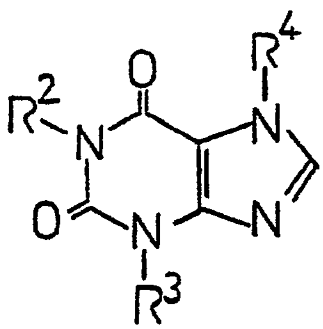

(II)

in der $R^2$, $R^3$ und $R^4$ unabhängig voneinander Wasserstoff oder Methylgruppen sind und $R^4$ auch eine Gruppe $-CH_2-COOR^5$, in der $R^5$ Wasserstoff oder eine Alkylgruppe mit 1 bis 4 C-Atomen ist, oder eine Gruppe $-CH_2-CH(OH)-CH_2-N(CH_3)CH_2-CH_2-OH$ sein kann, oder 1,3,7,9-Tetramethylharnsäure und/oder deren physiologisch verträgliche Salze enthalten sind.

Bevorzugte Verbindungen der Formel I sind 4-Alkoxybenzoesäuren, in welchen $R^1$ eine verzweigte Alkylgruppe ist. Bevorzugt geeignete 4-Alkoxybenzoesäuren sind z. B. 4-Isooctyl-, 4-Isononyl-, 4-Isodecyl-, 4-Isotridecyl- und 4-Isooctadecyl-benzoesäure.

Gut geeignet sind auch 4-(4-Alkylbenzyloxy)-benzoesäuren mit einer verzweigten Alkylgruppe, z. B. 4-(4tert. Butylbenzyloxy)-benzoesäure.

Als physiologisch verträgliche Salze eignen sich vor allem die Alkali- und Erdalkalisalze, z. B. die Natrium-, Kalium-, Calcium- oder Magnesiumsalze und die Ammonium- und Alkanolammoniumsalze, z. B. das Monoethanolammoniumsalz, das Isopropanolammoniumsalz oder das Triethanolammoniumsalz.

Einige der 4-Alkoxy- oder 4-(4-Alkylbenzyloxy)-benzoesäuren sind literaturbekannt. Sie lassen sich allgemein durch Hydrolyse der entsprechenden 4-Alkoxy- oder 4-(4-Alkylbenzyloxy)-benzoesäureester, z. B. der Methyl- oder Ethylester, unter literaturbekannten Hydrolysebedingungen herstellen.

Die 4-Alkoxy- oder 4-(4-Alkylbenzyloxy)-benzoesäureester lassen sich ihrerseits leicht durch Alkylierung von p-Hydroxybenzoesäureestern mit Alkylhalogeniden, z. B. der Formel $R^1$-Cl oder Alkylsulfaten, z. B. der Formel $R^1$-$OSO_3Na$, wobei $R^1$ die für die Formel I angegebene Bedeutung hat, nach allgemein bekannten Verfahren herstellen.

Die Herstellung einiger literaturbekannter 4-Alkoxybenzoesäuren wird im Beispielteil beschrieben.

Bevorzugt geeignete Purinverbindungen sind Theophyllin, Theobromin und Coffein. Auch Theophyllin-7-essigsäure und deren Methyl- und Ethylester und Xantinol, d. h. 7-(2-Hydroxy-3- (2-hydroxyethyl)-methylamino -propyl)-theophyllin, Xanthin und die 1,3,7,9-Tetramethylharnsäure sind wirksam. Als physiologisch verträgliche Salze der Purinbasen können die Hydrochloride, Phosphate, Carbonate, Acetate, Citrate und die Salze anderer ebenfalls topisch wirksamer Säuren, z. B. das durchblutungsfördernde Nicotinat eingesetzt werden.

Die 4-Alkoxy- oder 4-(4-Alkylbenzyloxy)-benzoesäuren der Formel I werden bevorzugt in einer Menge von 0,001 bis 0,5 Gew.-% der Zubereitungen und die Purinverbindungen werden bevorzugt in einer Menge von 0,01 bis 5,0 Gew.-% eingesetzt. Das Gewichtsverhältnis von 4-Alkoxy- oder 4-(4-Alkylbenzoxy)-benzoesäuren zu Purinverbindungen beträgt in den erfindungsgemäßen Zubereitungen bevorzugt 1 : 10 bis 1 : 50.

Als kosmetische Träger eignen sich alle für die Aufbringung auf die Haare oder die Haut geeigneten Zubereitungen. Für die Hautbehandlung eignen sich insbesondere wäßrige oder alkoholische Lösungen, tensidhaltige Lotionen, Öle, Salben, Emulsionen, Cremes, Gele und Stiftpräparate. Für die Haarbehandlung eignen sich besonders Haarwässer, Haarshampoos, Haarkuren, Haarspülungen und Haarsprays. Wegen der besonderen kosmetischen Probleme, die durch fettendes Haar verursacht werden, stellen die haarkosmetischen Zubereitungen besonders bevorzugte Ausführungsformen der Erfindung dar.

Die erfindungsgemäße Kombination von 4-Alkoxy- oder 4-(4-Alkylbenzyloxy)-benzoesäuren und Purinverbindungen besitzt eine ausgeprägte sebosuppressive Wirkung und eine gute Haut- und Schleimhautverträglichkeit. Mit den in kosmetischen Zubereitungen üblichen Komponenten besteht eine gute Verträglichkeit, so daß die Kombination in die verschiedensten kosmetischen Zubereitungen ohne Schwierigkeiten eingearbeitet werden kann.

Die wichtigsten Komponenten üblicher kosmetischer Träger sind

- Ölkomponenten, z. B. Paraffinöl, Pflanzenöle, Fettsäureester, Squalan, Fettalkohole, 2-Octyldodecanol,
- Fette und Wachse, z. B. Walrat, Bienenwachs, Montanwachs, Paraffin, Cetyl-stearylalkohol,
- Emulgatoren, z. B. Fettsäurepartialglyceride, Fettsäure-Sorbitan-partialester und deren Ethoxylate, Seifen, Fettalkoholsulfate, Fettalkoholpolyglycolether, Alkylphosphate,
- Waschrohstoffe, insbesondere Aniontenside, z. B. Fettalkoholpolyglycolethersulfate, Fettalkoholsulfate, Alphaolefinsulfonate, Alkansulfonate, Sulfobernsteinsäureester, Acyltauride, Acylisethionate und Acylsarkosine,

ampholytische Tenside, z. B. N-Alkylglycin, N-Alkylaminopropionsäure, N-Alkylaminobuttersäure mit 8 bis 18 C-Atomen in der Alkylgruppe, zwitterionische Tenside, z. B. N-Alkyl($C_8$-$C_{18}$)- N,N-dimethylammonio-glycinat oder N-Kokosacylaminopropyl-N,N-dimethylammonioglycinat und

nichtionogene Tenside, z. B. Fettalkoholpolyglycolether, Alkylphenolpolyglycolether, Fettsäurepolyglycolester, Aminoxid-Tenside, Fettsäurealkanolamide und deren Ethoxylate und

kationische Tenside, z. B. Alkyl($C_{12}$-$C_{18}$)-trimethylammoniumchlorid, Lauryldimethylbenzylammoniumchlorid, Cetylpyridiniumchlorid, Distearyldimethylammoniumchlorid

- niedere Alkohole wie z. B. Ethanol, Isopropanol,
- mehrwertige Alkohole wie z. B. Ethylenglycol, Propylenglycol, Glycerin,
- Wasser und Hilfsstoffe wie z. B. Duftstoffe, Konservierungsmittel, Puffersubstanzen, Verdickungsmittel, Farbstoffe und Trübungsmittel.

Die folgenden Beispiele sollen den Erfindungsgegenstand näher erläutern:

## Beispiele

1. Herstellungsbeispiele für 4-Alkoxy-benzoesäuren

A) 4-Tetradecyloxy-benzoesäure

20 g Tetradecyloxy-benzoesäuremethylester wurden mit 2,75 g Natriumhydroxid in 80 ml Ethanol gelöst und nach Zusatz von 50 ml Wasser 2,5 Stunden zum Sieden erhitzt. Nach dem Eindampfen der Suspension wurde in heißem Wasser gelöst und die Lösung mit verdünnter Salzsäure angesäuert. Nach Abfiltrieren, Waschen mit Wasser und Trocknen wurden 18,2 g (95 % d. Th.) 4-Tetradecyloxy-benzoesäure von Schmelzpunkt 95 bis 99 °C (klar bei 135 °C) erhalten.

Analog wurden die folgenden Säuren erhalten:

B) 4-Dodecyloxy-benzoesäure Schmelzpunkt 94 bis 95 °C (klar bei 135 °C)

C) 4-Decyloxy-benzoesäure Schmelzpunkt 97 bis 98 °C (klar bei 120 °C)

D) 4-Isononyloxy-benzoesäure (neue Verbindung) Schmelzpunkt 118 bis 119 °C

E) 4-(4-tert.Butyl-benzyloxy)-benzoesäure (neue Verbindung) Schmelzpunkt 236 bis 239 °C

F) 4-Isotridecyloxy-benzoesäure (neue Verbindung) Schmelzpunkt 56 °C (ab 36 °C Sintern)

2. Prüfung und Bewertung der antiseborrhoischen Wirkung

2.1 Grundlage

Der Test geht von der Beobachtung aus, daß männliche Ratten ein bräunliches Hautfett absondern, so daß die mehr oder weniger starke Fettigkeit der Haut visuell gut als Hautbräunung beurteilt werden kann. Daß es sich bei der Bräunung um Hautoberflächenfett handelt, ist daran zu erkennen, daß junge weibliche Ratten sowie männliche Ratten nach dem Waschen mit Tensidlösungen oder mit Lipidlösungsmitteln oder auch männliche Ratten, die systematisch mit Östrogen behandelt wurden, nur die normale helle, rosafarbene Haut nach dem Scheren aufweisen. Parallel dazu sind aus den abgeschnittenen Haaren nur noch sehr geringe Lipidmengen zu extrahieren.

2.2 Durchführung

Als Versuchstiere dienten männliche Wistar-Ratten mit einem Körpergewicht von 220 bis 230 g zu Versuchsbeginn.

Zur Beurteilung der Wirksamkeit wurden die Prüfsubstanzen in den in Tabelle 1 angegebenen Konzentrationen in Ethanol/Aceton (1 : 1) jeweils 6 Ratten halbseitig auf das Rückenfell gepinselt. Die andere Seite wurde nur mit dem Lösungsmittel behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert. Dabei wurde der Bräunungsgrad auf dem Rücken der Ratten als Maß für den Hautfettbelag visuell beurteilt.

2.3 Bewertung

Als 1. Kriterium wurde der Unterschied zwischen rechter und linker Seite gewertet, wobei pro Beurteiler und Tier jeweils 1 Punkt zu vergeben war, und zwar in der Weise, daß die
dunklere Seitemit 1 Punkt
hellere Seitemit 0 Punkten und
bei Gleichheit beide Seitenmit 0,5 Punkten
benotet wurden.

Signifikante Differenzen zwischen unbehandelter und behandelter Seite nach dieser Bewertungsmethode zeigen die lokale Wirksamkeit einer Substanz an.

Als 2. Kriterium wurden außerdem noch die Intensitätsunterschiede der Brauntöne nach folgender Skala bewertet:
3 Punktestark braun
2 Punktemittel braun
1 Punktschwach braun
0 Punktekeine Braunfärbung.

Nach dieser Bewertungsmethode werden die Punktsummendifferenzen zwischen den unbehandelten Kontrolltieren und jeweils den behandelten und unbehandelten Seiten (Δ P) der Versuchstiere gebildet, wobei wiederum signifikante Differenzen zwischen Kontrolltieren und der behandelten Seite der Versuchstiere die Wirkung einer Substanz deutlich machen.

Prozentuale Sebumreduktion

Die Sebumreduktion errechnet sich aus der Punktedifferenz in der Weise, daß man den Quotienten aus der Punktedifferenz Δ P und der Punktezahl für die Kontrollgruppe $P_K$ bildet und den erhaltenen Wert in % angibt.

$$\text{Sebumreduktion} = \frac{\Delta P}{P_k} \cdot 100 \quad (\%)$$

Tabelle I

| | Gew.-% | Gew.-% | Gew.-% | Gew.-% | Gew.-% | Gew.-% | Gew.-% | Gew.-% | Gew.-% | Gew.-% | Gew.-% |
|---|---|---|---|---|---|---|---|---|---|---|---|
| 4-Dodecyloxybenzoesäure | 0,01 | - | - | - | - | - | - | - | - | 0,01 | - |
| 4-Isononyloxybenzoesäure | - | 0,02 | - | - | - | - | 0,02 | - | - | - | - |
| 4-Isotridecyloxybenzoesäure | - | - | 0,002 | 0,001 | - | - | - | 0,002 | 0,002 | - | 0,001 |
| Theophyllin | - | - | - | - | 0,2 | - | 0,2 | 0,2 | 0,05 | - | - |
| Coffein | - | - | - | - | - | 0,1 | - | - | - | 0,1 | 0,1 |
| Ethanol/Aceton (1 : 1) | ad 100 | ad 100 | ad 100 | ad 100 | ad 100 | ad 100 | ad 100 | ad 100 | ad 100 | ad 100 | ad 100 |
| Prozentuale Sebumreduktion | 0 | 27 | 10 | 0 | 0 | 0 | 74 | 39 | 20 | 49 | 48 |

3. Rezepturbeispiele

3.1 Shampoo für fettendes Haar

| | | |
|---|---|---|
| Texapon ® N 25 (1) | 40 | Gew.-% |
| Comperlan ® KD (2) | 3 | |
| 4-Isononyloxy-benzoesäure | 0,04 | |
| Theophyllin | 0,2 | |
| Bronidox ® L (3) | 0,2 | |
| Wasser | ad 100 | |

3.2 Schnellhaarkur-Emulsion

| | | |
|---|---|---|
| Cetylalkohol | 3,0 | Gew.-% |
| Dehyquart ® A (4) | 2,0 | |
| 4-Isotridecyloxy-benzoesäure | 0,02 | |
| Theobromin | 0,2 | |
| Citronensäure | 1,0 | |
| Wasser | ad 100 | |

3.3 Schnellhaarkur, klar

| | | |
|---|---|---|
| Cetiol ® HE (5) | 20,0 | Gew.-% |
| Cetylpyridiniumchlorid | 5,0 | |
| Glycerin | 5,0 | |
| 4-Dodecyloxy-benzoesäure | 0,05 | |
| Coffein | 0,1 | |
| Isopropanol | ad 100 | |

3.4 Haarwasser

| | | |
|---|---|---|
| Cetiol ® HE (5) | 2,0 | Gew.-% |
| Birkenextrakt | 1,0 | |
| 4-Isononyloxy-benzoesäure | 0,005 | |
| Theophyllin | 0,1 | |
| Isopropanol | 30,0 | |
| Wasser | ad 100 | |

3.5 Hautemulsion O/W

| | | | |
|---|---|---|---|
| Cutina ® MD | (6) | 7,0 | Gew.-% |
| Eumulgin ® B1 | (7) | 3,0 | |
| Cetiol ® SN | (8) | 10,0 | |
| Myritol ® 318 | (9) | 10,0 | |
| 4-Isononyloxy-benzoesäure | | 0,01 | |
| Theophyllin | | 0,1 | |
| Wasser | | ad 100 | |

3.6 Hautcreme O/W

| | | | |
|---|---|---|---|
| Cutina ® MD | (6) | 17 | Gew.-% |
| Eumulgin ® B1 | (7) | 3 | |
| Eutanol ® G | (10) | 11 | |
| Myritol ® 318 | (9) | 6 | |
| Karottenöl CLR | | 3 | |
| 4-Isotridecyloxy-benzoesäure | | 0,01 | |
| Theophyllin | | 0,1 | |
| Wasser | | ad 100 | |

Die in den Rezepturbeispielen verwendeten Handelsnamen haben folgende Bedeutung:

(1) Texapon ® N 25:28%ige wäßrige Lösung von Alkyl-($C_{12}$-$C_{14}$)poly(2 EO)glycolethersulfat-Na-Salz
(2) Comperlan ® KD:Kokosfettsäurediethanolamid
(3) Bronidox ® L:5-Brom-5-nitro-1,3-dioxan (10%ige Lösung in 1,2-Propylenglycol)
(4) Dehyquart ® A:Cetyltrimethylammoniumchlorid (25%ige Lösung in Wasser)
(5) Cetiol ® HE:Polyol-Fettsäureester (CTFA-Bezeichnung: PEG-7-Glyceryl-Cocoate)
(6) Cutina ® MD:Palmitin/stearinsäure-mono/diglycerid
(7) Eumulgin ® B1:Cetyl/stearylalkohol + 12 Mol Ethylenoxid
(8) Cetiol ® SN:Cetyl/stearyl-isononanoat
(9) Myritol ® 318:Capryl/caprinsäure-triglycerid
(10) Eutanol ® G:2-Octyldodecanol

## Patentansprüche

1. Sebosuppressive Zubereitungen zur topischen Anwendung auf dem Haar und auf der Haut, bestehend aus antiseborrhoischen Wirkstoffen und einem kosmetischen Träger, dadurch gekennzeichnet, daß als antiseborrhoische Wirkstoffe eine synergistisch wirkende Kombination aus

(a) 4-Alkoxy- oder 4-(4-Alkylbenzyloxy)-benzoesäuren der Formel I

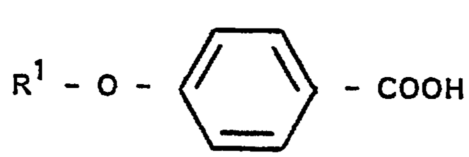

(I)

in der $R^1$ eine Alkylgruppe mit 6 bis 18 C-Atomen oder eine 4-Alkylbenzylgruppe mit 4 bis 12 C-Atomen in der Alkylgruppe ist oder deren physiologisch verträgliche Salze und

(b) Purinverbindungen der allgemeinen Formel II

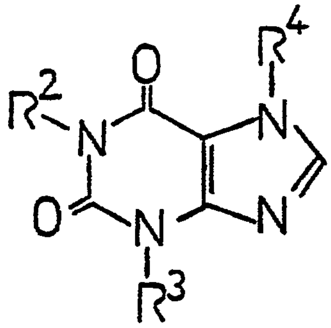

(II)

in der $R^2$, $R^3$ und $R^4$ unabhängig voneinander Wasserstoff oder Methylgruppen sind und $R^4$ auch eine Gruppe $-CH_2-COOR^5$, in der $R^5$ Wasserstoff oder eine Alkylgruppe mit 1 bis 4 C-Atomen ist, oder eine Gruppe
$-CH_2-CH(OH)-CH_2-N(CH_3)CH_2-CH_2-OH$
ist oder 1,3,7,9-Tetramethylharnsäure und/oder deren physiologisch verträgliche Salze enthalten ist.

2. Sebosuppressive Zubereitungen nach Anspruch 1, dadurch gekennzeichnet, daß die Verbindungen der Formel I ausgewählt sind aus 4-Isooctyl-benzoesäure, 4-Isononyl-benzoesäure, 4-Isodecyl-benzoesäure, 4-Isotridecyl-benzoesäure, 4-Isooctadecyl-benzoesäure, 4-Decyloxy-benzoesäure und 4-Dodecyloxy-benzoesäure.

3. Sebosuppressive Zubereitungen nach den Ansprüchen 1 oder 2, dadurch gekennzeichnet, daß als Purinverbindungen Theophyllin, Theobromin oder Coffein enthalten sind.

4. Sebosuppressive Zubereitungen nach den Ansprüchen 1 bis 3, dadurch gekennzeichnet, daß die Verbindungen der Formel I in einer Menge von 0,001 bis 0,5 Gew.-% und die Purinverbindungen in einer Menge von 0,01 bis 5,0 Gew.-%, bezogen auf die gesamte Zubereitung enthalten sind.

## Claims

1. Sebosuppressive preparations for topical application to the hair and to the skin, consisting of antiseborrheic agents and a cosmetic carrier, characterized in that they contain as antiseborrheic agents a synergistically acting combination of

(a) 4-alkoxy- or 4-(4-alkylbenzyloxy)-benzoic acids corresponding to the following formula

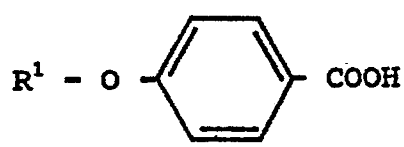

in which $R^1$ is a $C_{6-18}$ alkyl group or a 4-alkylbenzyl group containing from 4 to 12 C atoms in the alkyl groups,
or physiologically acceptable salts thereof and

(b) purine compounds corresponding to the following general formula

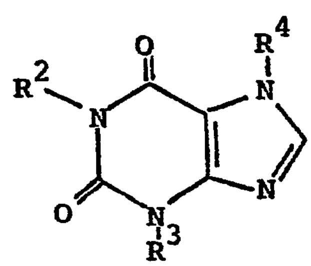

in which $R^2$, $R^3$ and $R^4$ independently of one another represent hydrogen or methyl groups and $R^4$ may also be a group $-CH_2COOR^5$, in which $R^5$ is hydrogen or a $C_{1-4}$ alkyl group, or a group
$-CH_2-CH(OH)-CH_2-N(CH_3)CH_2-CH_2-OH$,
or 1,3,7,9-tetramethyl uric acid and/or physiologically acceptable salts thereof.

2. Sebosuppressive preparations as claimed in claim 1, characterized in that the compounds of formula I are selected from 4-isooctylbenzoic acid, 4-isononylbenzoic acid, 4-isodecylbenzoic acid, 4-isotri-

decylbenzoic acid, 4-isooctadecylbenzoic acid, 4-decyloxybenzoic acid and 4-dodecyloxybenzoic acid.

3. Sebosuppressive preparations as claimed in claim 1 or 2, characterized in that they contain theophylline, theobromine or caffeine as purine compounds.

4. Sebosuppressive preparations as claimed in claims 1 to 3, characterized in that the compounds of formula I are present in a quantity of from 0.001 to 0.5% by weight while the purine compounds are present in a quantity of from 0.01 to 5.0% by weight, based on the preparation as a whole.

**Revendications**

1. Compositions anti-séborrhéiques pour application locale sur le cheveux et la peau, constituées de substances actives anti-séborrhéiques et d'un véhicule cosmétique, caractérisées en ce qu'elles contiennent en tant que substances actives antiséborrhéiques une combinaison synergique active

a) des acides 4-alcoxy- ou 4-(4-alkylbenzyloxy)-benzoïques de formule I:

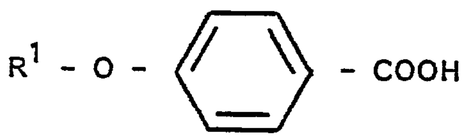

(I)

dans laquelle $R^1$ est un groupe alkyle ayant de 6 à 18 atomes de carbone ou un groupe 4-alkylbenzyle ayant de 4 à 12 atomes de carbone dans le fragment alkyle, ou leurs sels physiologiquement acceptables, et

b) des composés puriques de formule générale II:

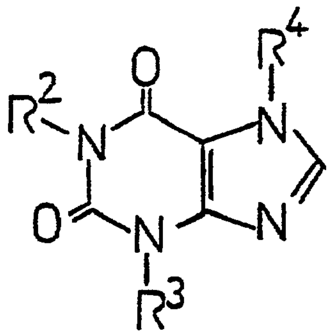

(II)

dans laquelle $R^2$, $R^3$ et $R^4$ sont indépendamment les uns des autres des atomes d'hydrogène ou des groupes alkyle et $R^4$ peut être également un groupe $-CH_2-COOR^5$, dans lequel $R^5$ est un atome d'hydrogène ou un groupe alkyle ayant de 1 à 4 atomes de carbone, ou un groupe $-CH_2-CH(OH)-CH_2-N(CH_3)CH_2-CH_2-OH$, ou l'acide 1,3,7,9-tétraméthylurique et/ou ses sels physiologiquement acceptables.

2. Compositions antiséborrhéiques selon la revendication 1, caractérisées en ce que les composés de formule I sont choisis parmi l'acide 4-isooctylbenzoïque, l'acide 4-isononyl-benzoïque, l'acide 4-isododécyl-benzoïque, l'acide 4-isotrodécyl-benzoïque, l'acide 4-iso-octadécyl-benzoïque, l'acide 4-décyloxy-benzoïque et l'acide 4-dodécyloxy-benzoïque.

3. Compositions antiséborrhéiques selon la revendication 1 ou 2, caractérisées en ce qu'elles contiennent en tant que composés puriques de la théophylline, de la théobromine ou de la caféine.

4. Compositions antiséborrhéiques selon l'une quelconque des revendications 1 à 3, caractérisées en ce que les composés de formule I sont contenus en une quantité de 0,001 à 0,5% en poids et les composés puriques sont contenus en quantité de 0,01 à 5,0% en poids, par rapport à la composition totale.